# FASCICULE DE BREVET EUROPEEN

(11) **EP 2 090 515 B1**
(45) Date de publication et mention de la délivrance du brevet: **15.06.2011**
(21) Numéro de dépôt: 09152917.2
(22) Date de dépôt: 16.02.2009
(51) Int. Cl.: B65D 1/08, B65D 47/10, B65D 47/18, A61F 9/00

(54) **Flacon sécable destiné à contenir un liquide**
Teilbarer Flakon zur Aufnahme einer Flüssigkeit
Divisible bottle designed to contain a liquid

(30) Priorité: 18.02.2008 FR 0800854
(43) Date de publication de la demande: 19.08.2009
(73) Titulaire: Seriplast, 01100 Oyonnax (FR)
(72) Inventeur: Tartaglione, André, 01100 Oyonnax (FR)
(74) Mandataire: Goreaud, Alexandra

(56) Documents cités:
- DE-A1- 4 125 987
- DE-U1- 9 115 539
- GB-A- 2 135 290

## Description

La présente invention concerne un flacon sécable destiné à contenir un liquide.

De tels flacons sont généralement utilisés dans le domaine pharmaceutique ou cosmétique. Il s'agit de flacons le plus souvent à usage unique, qui doivent être cassés avant la première utilisation afin de libérer le liquide qu'ils contiennent. Voir par exemple GB 2 135 290 A.

Toutefois, une fois le flacon cassé, l'ouverture créée pour l'écoulement du liquide ne convient pas à toutes les utilisations. En effet, cette ouverture présente généralement un bord tranchant, possédant des aspérités, si bien que le produit ne peut pas être versé sur des zones sensibles de la peau, ou dans l'oeil, par exemple. De plus, si l'ouverture présente des dimensions relativement importantes, une application précise sur une zone très localisée peut être problématique.

Pour palier ce problème, il est connu de prévoir un dispositif de distribution du liquide distinct du flacon, que l'utilisateur va venir fixer dans le flacon une fois que ce dernier aura été cassé. Cette solution présente cependant des inconvénients. Ainsi, d'une part, la stérilité du liquide ne peut être garantie puisqu'on introduit une pièce étrangère dans le flacon, qui aura de plus été en contact avec les doigts de l'utilisateur. D'autre part, la mise en oeuvre de ce type de flacon est peu commode, car il faut assembler plusieurs pièces avant de pouvoir verser le liquide. Ceci est d'autant plus malaisé que les pièces sont fragiles et/ou de petites dimensions.

La présente invention vise à remédier aux inconvénients mentionnés ci-dessus.

A cet effet, l'invention concerne un flacon sécable destiné à contenir un liquide et comportant :
- un corps réalisé d'une seule pièce, présentant une partie principale dont l'extrémité amont est ouverte et une partie formant capuchon qui prolonge la partie principale vers l'aval, avant la première utilisation, et dont l'extrémité aval est fermée, une zone de rupture étant ménagée sensiblement à la jonction entre la partie principale et le capuchon ;
- une pièce formant fond, distincte du corps et solidarisée à l'extrémité amont dudit corps de façon étanche.

Le flacon est plus particulièrement caractérisé en ce qu'il comporte en outre un dispositif de distribution du liquide comprenant d'une part une base agencée pour être fixée avec étanchéité dans la partie principale du corps et d'autre part un tube creux, lié à la base, qui s'étend à l'intérieur du capuchon avant la rupture du corps, et qui est destiné à permettre l'écoulement du liquide contenu dans le flacon vers l'extérieur une fois que le corps a été rompu au niveau de la zone de rupture, ledit tube étant élastiquement déformable, de sorte qu'il puisse être courbé sans casser lors de la rupture du corps.

Les termes « amont » et « aval » sont définis par rapport au sens d'écoulement du liquide vers l'extérieur du flacon. Par « étanche », on entend une étanchéité au liquide contenu dans le flacon.

Ainsi, l'invention fournit un flacon prêt à l'emploi, qui permet une application du liquide en toute sécurité grâce au dispositif de distribution intégré, sans risque de perte d'hygiène ou de stérilité. L'utilisateur a simplement à casser le flacon avant la première utilisation, et peut ensuite verser le liquide à l'endroit désiré, sans que ce liquide ne soit au contact des doigts de l'utilisateur ou d'un élément potentiellement sali lors des manipulations préalables à la distribution du produit.

Le dispositif de distribution du liquide est par exemple un dispositif de type goutte à goutte.

Selon une réalisation possible, la partie principale du corps comprend une première portion cylindrique et une deuxième portion cylindrique, située en aval de la première portion cylindrique et de plus petit diamètre, la base du dispositif de distribution présentant une partie cylindrique de diamètre extérieur sensiblement égal au diamètre intérieur de ladite deuxième portion cylindrique et étant montée de façon étanche dans ladite deuxième portion cylindrique.

En outre, la base du dispositif de distribution peut comporter une partie tronconique convergeant vers l'aval qui prolonge vers l'aval la partie cylindrique, et la deuxième portion cylindrique de la partie principale du corps peut être prolongée vers l'aval par une portion tronconique convergeant vers l'aval, dont la forme intérieure est complémentaire de la forme extérieure de la partie tronconique de la base.

La zone de rupture du corps est alors située à l'extrémité aval de la portion tronconique de la partie principale du corps.

On décrit à présent, à titre d'exemple non limitatif, un mode de réalisation possible de l'invention, en référence aux figures annexées :
Les figures 1 à 3 représentent respectivement le corps du flacon, le fond du flacon et le dispositif de distribution de liquide, avant l'assemblage de ces trois pièces ;
La figure 4 est une vue en coupe longitudinale du flacon assemblé, avant la première utilisation ;
La figure 5 est une vue en coupe longitudinale du flacon, lors de la rupture du corps ; et
La figure 6 est une vue en coupe longitudinale du flacon prêt à l'emploi, après que le corps a été rompu.

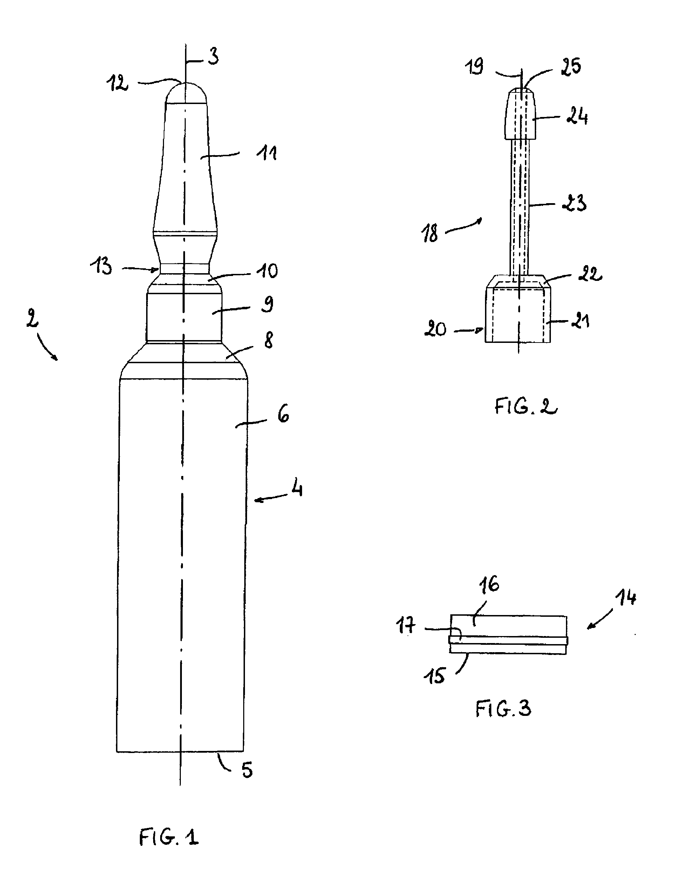

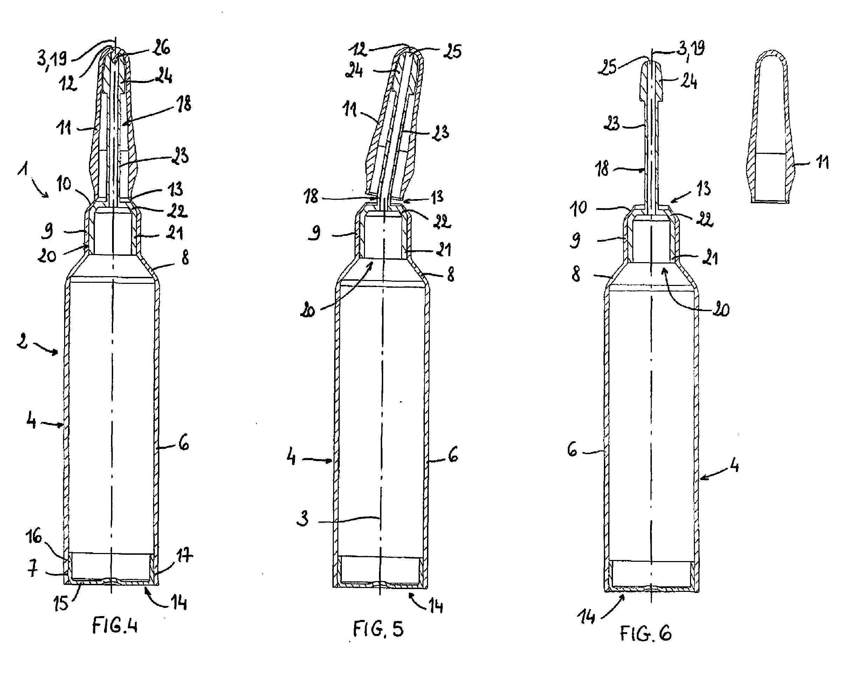

Comme illustré sur les figures 1 et 4, un flacon 1 comprend tout d'abord un corps 2 présentant un axe 3. Le corps 2 est réalisé d'une seule pièce, par exemple par moulage d'une matière plastique telle que le polypropylène. En variante, le corps 2 pourrait être en verre.

Le corps 2 comprend une partie principale 4 possédant une extrémité amont 5 ouverte. Dans la réalisation représentée, cette partie principale 4 comporte successivement, de l'amont vers l'aval :
- une première portion cylindrique 6, s'étendant sur au moins les trois quarts de la partie principale 4 et présentant, à proximité de l'extrémité amont 5, une gorge annulaire 7 ;
- une première portion tronconique 8 convergeant vers l'aval ;
- une deuxième portion cylindrique 9 de plus petit diamètre que la première portion cylindrique 6 ;
- et une deuxième portion tronconique 10 convergeant vers l'aval.

L'épaisseur de la paroi de la partie principale 4 est sensiblement constante dans toutes ces portions.

Le corps 2 comprend également une partie formant capuchon 11 qui prolonge la partie principale 4 vers l'aval, avant la première utilisation, et qui possède une extrémité aval 12 fermée.

La jonction entre la partie principale 4 et le capuchon 11 forme une zone de rupture 13 permettant de casser le corps 2 en deux parties. Selon une réalisation possible, la zone de rupture 13 est formée par un amincissement localisé de la paroi du corps 2.

Le flacon 1 comprend également un fond 14, représenté sur les figures 3 et 4. Le fond 14 possède une paroi transversale 15 en forme de disque et une jupe 16 cylindrique munie d'un bourrelet annulaire 17.

Enfin, le flacon 1 comprend un dispositif de distribution 18 du liquide, plus spécifiquement illustré sur la figure 2. Le dispositif de distribution 18 présente un axe 19 et comprend une base 20, creuse et ouverte à son extrémité amont, possédant une partie cylindrique 21 de diamètre extérieur sensiblement égal au diamètre intérieur de la deuxième portion cylindrique 9 de la partie principale 4 du corps 2. La partie cylindrique 21 est prolongée, vers l'aval, par une partie tronconique 22 convergeant vers l'aval, dont la forme extérieure est complémentaire de la forme intérieure de la deuxième portion tronconique 10 de la partie principale 4 du corps 2.

Le dispositif de distribution 18 comprend de plus un tube 23 creux, lié à la base 20 et de même axe 19 que celle-ci. Les dimensions et le matériau constitutif du tube 23 lui permettent d'avoir une certaine souplesse pour pouvoir être déformé élastiquement dans les conditions d'utilisation. Par exemple, le tube 23 est réalisé en polyéthylène. Une tête 24 est ménagée à la partie extrême aval du tube 23, ladite tête 24 possédant une forme extérieure complémentaire de la forme intérieure du capuchon 11. Dans la réalisation représentée, la tête 24 présente la forme d'un obus à l'extrémité arrondie.

Avantageusement, le dispositif de distribution 18 peut être réalisé d'une seule pièce, par exemple par moulage d'une matière plastique telle que le polyéthylène.

Le flacon 1 est obtenu de la façon suivante.

Tout d'abord, le dispositif de distribution 18 est inséré sensiblement axialement dans le corps 2. La base 20 vient se positionner dans la deuxième portion cylindrique 9, en butée contre la deuxième portion tronconique 10. Ainsi, la base 20 est fixée dans la partie principale 4 du flacon 1 sensiblement immédiatement en amont de la zone de rupture 13. Dans cette position, la base 20 est fixée avec étanchéité dans la partie principale 4 du corps 2 du flacon 1 et le tube 23 s'étend à l'intérieur du capuchon 11, sensiblement depuis la zone de rupture 13 jusqu'à l'extrémité aval 12 du capuchon 11. Avantageusement, le corps 2 du flacon 1 et la base 20 du dispositif de distribution 18 peuvent posséder des moyens d'encliquetage réciproques permettant un excellent maintien.

Le corps 2 est alors rempli du liquide, depuis son extrémité amont 5 ouverte. Par exemple, le flacon a une capacité d'environ 11 ml.

Enfin, après remplissage du flacon, le fond 14 est encliqueté dans le corps 2 depuis l'extrémité amont 5, le bourrelet 17 coopérant avec la gorge 7 pour assurer la retenue du fond 14 et l'étanchéité du flacon 1.

Le flacon ainsi assemblé et rempli, avant sa première utilisation c'est-à-dire avant la rupture du corps 2, est illustré sur la figure 4.

De préférence, dans cette position, le dispositif de distribution 18 et le capuchon 11 sont agencés pour coopérer afin d'empêcher la sortie du liquide par l'extrémité aval 25 du tube 23. A cet effet, les dispositions suivantes sont possibles :
- l'extrémité aval 25 du tube 23 est en contact étanche avec l'extrémité aval 12 du capuchon 11. En d'autres termes, la face intérieure du capuchon 11 obture l'orifice aval du tube 23, cette face intérieure étant dépourvue d'organe en saillie (cas des figures 5 et 6) ;
- et/ou le capuchon 11 possède à son extrémité aval 12 un téton 26 faisant saillie vers l'intérieur et engagé dans le tube 23 de façon étanche (cas de la figure 4).

Lorsqu'un utilisateur souhaite verser le liquide contenu dans le flacon 1, il doit tout d'abord casser le corps 2, comme illustré sur la figure 5. Saisissant le capuchon 11 avec les doigts, l'utilisateur casse le corps 2 sensiblement au niveau de la zone de rupture 13, séparant ainsi le capuchon 11 et la partie principale 4 du corps 2. Au cours de cette opération, le capuchon 11 est incliné par rapport à la partie principale 4 et le tube 23 du dispositif de distribution 18, qui est toujours logé à l'intérieur du capuchon 11, se courbe sans se casser. Dans le cas où le téton 26 est présent, celui-ci ne gêne ni la rupture du corps 2 ni la flexion du tube 23. Il doit bien être compris que, lorsqu'il existe, ce téton 26 reste solidaire du capuchon 11 pendant cette opération de rupture et ultérieurement, les figures 5 et 6 illustrant une réalisation sans téton, différente de la réalisation avec téton de la figure 4.

Une fois le capuchon 11 ôté (figure 6), le tube 23 retrouve sa position droite (retour élastique) et l'utilisateur peut faire s'écouler le liquide contenu dans le flacon 1.

Selon une réalisation possible de l'invention, le capuchon 11 peut ensuite être replacé sur la partie principale 4 du corps 2 afin de l'obturer avec étanchéité, pour conserver le liquide contenu dans le flacon 1 en vue d'une utilisation ultérieure. En variante, le flacon selon l'invention peut être à usage unique et donc jeté une fois qu'il est vide.

Il va de soi que l'invention n'est pas limitée au mode de réalisation décrit ci-dessus à titre d'exemple mais qu'elle en embrasse au contraire toutes les variantes de réalisation.

## Revendications

1. Flacon sécable destiné à contenir un liquide et comportant :
- un corps (2) réalisé d'une seule pièce, présentant une partie principale (4) dont l'extrémité amont (5) est ouverte et une partie formant capuchon (11) qui prolonge la partie principale (4) vers l'aval, avant la première utilisation, et dont l'extrémité aval (12) est fermée, une zone de rupture (13) étant ménagée sensiblement à la jonction entre la partie principale (4) et le capuchon (11) ;
- une pièce formant fond (14), distincte du corps (2) et solidarisée à l'extrémité amont (5) dudit corps (2) de façon étanche ;
**caractérisé en ce qu'**il comporte en outre un dispositif de distribution (18) du liquide comprenant d'une part une base (20) agencée pour être fixée avec étanchéité dans la partie principale (4) du corps (2) et d'autre part un tube (23) creux, lié à la base (20), qui s'étend à l'intérieur du capuchon (11) avant la rupture du corps (2), et qui est destiné à permettre l'écoulement du liquide contenu dans le flacon (1) vers l'extérieur une fois que le corps (2) a été rompu au niveau de la zone de rupture (13), ledit tube (23) étant élastiquement déformable, de sorte qu'il puisse être courbé sans casser lors de la rupture du corps (2).

2. Flacon selon la revendication 1, **caractérisé en ce que** le dispositif de distribution (18) et le capuchon (11) sont agencés pour coopérer, avant la rupture du corps (2), afin d'empêcher la sortie du liquide par l'extrémité aval (25) du tube (23).

3. Flacon selon la revendication 1 ou 2, **caractérisé en ce que**, avant la première utilisation, l'extrémité aval (25) du tube (23) est en contact étanche avec l'extrémité aval (12) du capuchon (11).

4. Flacon selon l'une des revendications 1 à 3, **caractérisé en ce que** le capuchon (11) possède à son extrémité aval (12) un téton (26) faisant saillie vers l'intérieur et engagé dans le tube (23) creux de façon étanche.

5. Flacon selon l'une des revendications 1 à 4, **caractérisé en ce que** le dispositif de distribution (18) comporte une tête (24) ménagée à la partie extrême aval du tube (23), ladite tête (24) possédant une forme extérieure complémentaire de la forme intérieure du capuchon (11).

6. Flacon selon l'une des revendications 1 à 5, **caractérisé en ce que** la partie principale (4) du corps (2) comprend une première portion cylindrique (6) et une deuxième portion cylindrique (9), située en aval de la première portion cylindrique (6) et de plus petit diamètre, la base (20) du dispositif de distribution (18) présentant une partie cylindrique (21) de diamètre extérieur sensiblement égal au diamètre intérieur de ladite deuxième portion cylindrique (9) et étant montée de façon étanche dans ladite deuxième portion cylindrique (9).

7. Flacon selon la revendication 6, **caractérisé en ce que** la base (20) du dispositif de distribution (18) comporte une partie tronconique (22) convergeant vers l'aval qui prolonge vers l'aval la partie cylindrique (21), et **en ce que** la deuxième portion cylindrique (9) de la partie principale (4) du corps (2) est prolongée vers l'aval par une portion tronconique (10) convergeant vers l'aval, dont la forme intérieure est complémentaire de la forme extérieure de la partie tronconique (22) de la base (20).

8. Flacon selon la revendication 7, **caractérisé en ce que** la zone de rupture (13) du corps (2) est située à l'extrémité aval de ladite portion tronconique (10) de la partie principale (4) du corps (2).

9. Flacon selon l'une des revendications 1 à 8, **caractérisé en ce que** le corps (2) du flacon (1) et la base (20) du dispositif de distribution (18) possèdent des moyens d'encliquetage réciproques.

10. Flacon selon l'une des revendications 1 à 9, **caractérisé en ce que** la zone de rupture (13) du corps (2) est formée par un amincissement localisé de la paroi du corps (2).

## Claims

1. A breakable flask intended to contain a liquid and including:
- a body (2) made in a single piece, having a main portion (4), the upstream end (5) of which is open, and a portion forming a cap (11), which extends the main portion (4) downstream, before first use, and the downstream end (12) of which is closed, a breaking area (13) being made substantially at the junction between the main portion (4) and the cap (11);
- a part forming a bottom (14) distinct from the body (2) and sealably secured to the upstream end (5) of said body (2);
**characterized in that** it further includes a device (18) for dispensing the liquid comprising a base (20) arranged so as to be sealably attached in the main portion (4) of the body (2) on the one hand and a hollow tube (23) bound to the base (20), which extends inside the cap (11) before breakage of the body (2), and which is intended to allow the liquid contained in the flask (1) to flow outwards once the body (2) has been broken at the breaking area (13), said tube (23) being elastically deformable are so that it may be bent without breaking when the body (2) is broken.

2. The flask according to claim 1, **characterized in that** the dispensing device (18) and the cap (11) are arranged in order to co-operate before breaking of the body (2), in order to prevent the liquid from flowing out through the downstream end (25) of the tube (23).

3. The flask according to claim 1 or 2, **characterized in that**, before first use, the downstream end (25) of the tube (23) is in sealed contact with the downstream end (12) of the cap (11).

4. The flask according to one of claims 1 to 3, **characterized in that** the cap (11) has at its downstream end (12) a nipple (26) protruding inwards and sealably engaged into the hollow tube (23).

5. The flask according to one of claims 1 to 4, **characterized in that** the dispensing device (18) includes a head (24) made at the downstream end portion of the tube (23), said head (24) having an outer shape mating the inner shape of the cap (11).

6. The flask according to one of claims 1 to 5, **characterized in that** the main portion (4) of the body (2) comprises a first cylindrical portion (6) and a second cylindrical portion (9) located downstream from the first cylindrical portion (6), and with a smaller diameter, the base (20) of the dispensing device (18) having a cylindrical portion (21) with an outer diameter substantially equal to the inner diameter of said second cylindrical portion (9) and sealably mounted in said second cylindrical portion (9).

7. The flask according to claim 6, **characterized in that** the base (20) of the dispensing device (18) includes a frusto-conical portion (22) converging downstream, which extends the cylindrical portion (21) downstream, and **in that** the second cylindrical portion (9) of the main portion (4) of the body (2) is extended downstream with a frusto-conical portion (10) converging downstream, the inner shape of which mates the outer shape of the frusto-conical portion (22) of the base (20).

8. The flask according to claim 7, **characterized in that** the breaking area (13) of the body (2) is located at the downstream end of said frusto-conical portion (10) of the main portion (4) of the body (2).

9. The flask according to one of claims 1 to 8, **characterized in that** the body (2) of the flask (1) and the base (20) of the dispensing device (18) have mutual snap-on fastening means.

10. The flask according to one of claims 1 to 9, **characterized in that** the breaking area (13) of the body (2) is formed by localized thinning of the wall of the body (2).

## Patentansprüche

1. Teilbare Flasche, die dazu bestimmt ist, eine Flüssigkeit zu enthalten und aufweist:
- einen aus einem Stück hergestellten Körper (2), der einen Hauptabschnitt (4) aufweist, dessen oberstromiges Ende (5) geöffnet ist, und einen Abschnitt, der eine Kappe (11) bildet, der den Hauptabschnitt (4) vor der ersten Verwendung unterstromig verlängert, und dessen unterstromiges Ende (12) geschlossen ist, wobei ein Bruchbereich (13) etwa an der Verbindung zwischen dem Hauptabschnitt (4) und der Kappe (11) ausgebildet ist,
- einen Abschnitt, der den Boden (14) bildet, der sich vom Körper (2) unterscheidet und mit dem oberstromigen Ende (5) des Körpers (2) dicht verbunden ist,
**dadurch gekennzeichnet, dass** sie weiterhin eine Verteilungsvorrichtung (18) der Flüssigkeit aufweist, die einerseits eine Basis (20) umfasst, die ausgebildet ist, um dicht im Hauptabschnitt (4) des Körpers (2) befestigt zu sein, und andererseits ein hohles Rohr (23), das mit der Basis (20) verbunden ist, das sich vor dem Bruch des Körpers (2) innerhalb der Kappe (11) erstreckt und das dazu bestimmt ist, das Abfließen der in der Flasche (1) enthaltenen Flüssigkeit nach außen zu gestatten, sobald der Körper (2) auf Ebene des Bruchbereichs (13) zerbrochen wurde, wobei das Rohr (23) elastisch deformierbar ist, so dass es krümmbar ist, ohne beim Zerbrechen des Körpers (2) zerstört zu werden.

2. Flasche nach Anspruch 1, **dadurch gekennzeichnet, dass** die Verteilungsvorrichtung (18) und die Kappe (11) ausgebildet sind, um vor dem Zerbrechen des Körpers (2) zusammenzuarbeiten, um den Austritt der Flüssigkeit durch das unterstromige Ende (25) des Rohrs (23) zu verhindern.

3. Flasche nach Anspruch 1 oder 2, **dadurch gekennzeichnet, dass** das unterstromige Ende (25) des Rohrs (23) vor der ersten Verwendung im dichten Kontakt mit dem unterstromigen Ende (12) der Kappe (11) ist.

4. Flasche nach einem der Ansprüche 1 bis 3, **dadurch gekennzeichnet, dass** die Kappe (11) an ihrem unterstromigen Ende (12) einen Ansatz (26) besitzt, der nach innen hervorsteht und in das hohle Rohr (23) dicht eingebracht ist.

5. Flasche nach einem der Ansprüche 1 bis 4, **dadurch gekennzeichnet, dass** die Verteilungsvorrichtung (18) einen Kopf (24) aufweist, der am unterstromigen Ende des Rohrs (23) ausgebildet ist, wobei der Kopf (24) eine äußere Form besitzt, der zur inneren Form der Kappe (11) komplementär ist.

6. Flasche nach einem der Ansprüche 1 bis 5, **dadurch gekennzeichnet, dass** der Hauptabschnitt (4) des Körpers (2) einen ersten zylindrischen Teil (6) und einen zweiten zylindrischen Teil (9) umfasst, der sich unterstromig zum ersten zylindrischen Teil (6) befindet und einen kleineren Durchmesser hat, wobei die Basis (20) der Verteilungsvorrichtung (18) einen zylindrischen Teil (21) mit einem Außendurchmesser aufweist, der etwa dem Innendurchmesser des zweiten zylindrischen Teils (9) entspricht und dicht in dem zweiten zylindrischen Teil (9) montiert ist.

7. Flasche nach Anspruch 6, **dadurch gekennzeichnet, dass** die Basis (20) der Verteilungsvorrichtung (18) einen kegelstumpfförmigen Teil (22) aufweist, der in unterstromige Richtung zusammenläuft, der den zylindrischen Teil (21) in unterstromige Richtung verlängert, und dass der zweite zylindrische Teil (9) des Hauptabschnitts (4) des Körpers (2) in unterstromige Richtung durch einen kegelstumpfförmigen Teil (10) verlängert wird, der in unterstromige Richtung zusammenläuft, dessen Innenform zur Außenform des kegelstumpfförmigen Teils (22) der Basis (20) komplementär ist.

8. Flasche nach Anspruch 7, **dadurch gekennzeichnet, dass** sich der Bruchbereich (13) des Körpers (2) am unterstromigen Ende des kegelstumpfförmigen Teils (10) des Hauptabschnitts (4) des Körpers (2) befindet.

9. Flasche nach einem der Ansprüche 1 bis 8, **dadurch gekennzeichnet, dass** der Körper (2) der Flasche (1) und die Basis (20) der Verteilungsvorrichtung (18) gegenseitige Rastmittel besitzen.

10. Flasche nach einem der Ansprüche 1 bis 9, **dadurch gekennzeichnet, dass** der Bruchbereich (13) des Körpers (2) von einer lokalisierten Verschlankung der Wand des Körpers (2) gebildet wird.
